# EUROPÄISCHE PATENTSCHRIFT

(11) **EP 1 901 671 B1**
(45) Veröffentlichungstag und Bekanntmachung des Hinweises auf die Patenterteilung: **17.04.2013**
(21) Anmeldenummer: 06776104.9
(22) Anmeldetag: 30.06.2006
(51) Int. Cl.: A61B 17/80

(54) **OSTEOSYNTHESEPLATTE MIT SCHRÄG ZUR PLATTENEBENE VERLAUFENDEN DURCHGANGSÖFFNUNGEN**
OSTEOSYNTHESIS PLATE COMPRISING THROUGH-OPENINGS WHICH ARE INCLINED IN RELATION TO THE PLANE OF THE PLATE
PLAQUE D'OSTEOSYNTHESE PRESENTANT DES OUVERTURES DE PASSAGE INCLINEES PAR RAPPORT AU PLAN DE LA PLAQUE

(30) Priorität: 08.07.2005 DE 102005032026
(43) Veröffentlichungstag der Anmeldung: 26.03.2008
(73) Patentinhaber: Stryker Leibinger GmbH & Co. KG, 79111 Freiburg (DE)
(72) Erfinder: JACOBS, Fred, J., 0043 Pretoria (ZA)
(74) Vertreter: Röthinger, Rainer
(86) Internationale Anmeldenummer: PCT/EP2006/006365
(87) Internationale Veröffentlichungsnummer: WO 2007/006430

(56) Entgegenhaltungen:
- EP-A- 1 153 577
- WO-A-00/66012
- DE-A1- 19 636 733
- DE-U1- 9 208 234
- US-A- 5 549 612

## Beschreibung

### Gebiet der Erfindung

Die Erfindung betrifft eine Osteosyntheseplatte mit schräg zur Plattenebene verlaufenden Durchgangsöffnungen. Derartige Osteosyntheseplatten können zur Versorgung von Frakturen im Bereich des Kopfes und insbesondere zur Versorgung von Kieferfrakturen eingesetzt werden.

### Hintergrund der Erfindung

Osteosyntheseplatten zur Behandlung von Frakturen sind seit über 100 Jahren bekannt. Die gebräuchlichsten Osteosyntheseplatten besitzen eine lineare (oder langgestreckte) Gestalt und werden von mehreren senkrecht zur Plattenebene verlaufenden Durchgangsöffnungen durchsetzt. Zum Anbringen einer Osteosyntheseplatte an einem Knochen oder Knochenfragment werden Befestigungselemente (üblicherweise Knochenschrauben) durch die Durchgangsöffnungen hindurch in den Knochen oder das Knochenfragment eingebracht.

Für einzelne Indikationen hat es sich als zweckmäßig erwiesen, die Durchgangsöffnungen schräg zur Plattenebene auszubilden. Häufig hängt das Vorsehen von schräg zur Plattenebene verlaufenden Durchgangsöffnungen mit bestimmten anatomischen Gegebenheiten oder mit besonderen Erfordernissen wie dem Erzeugen von unter bestimmten Winkeln verlaufenden Kompressionskräften zusammen.

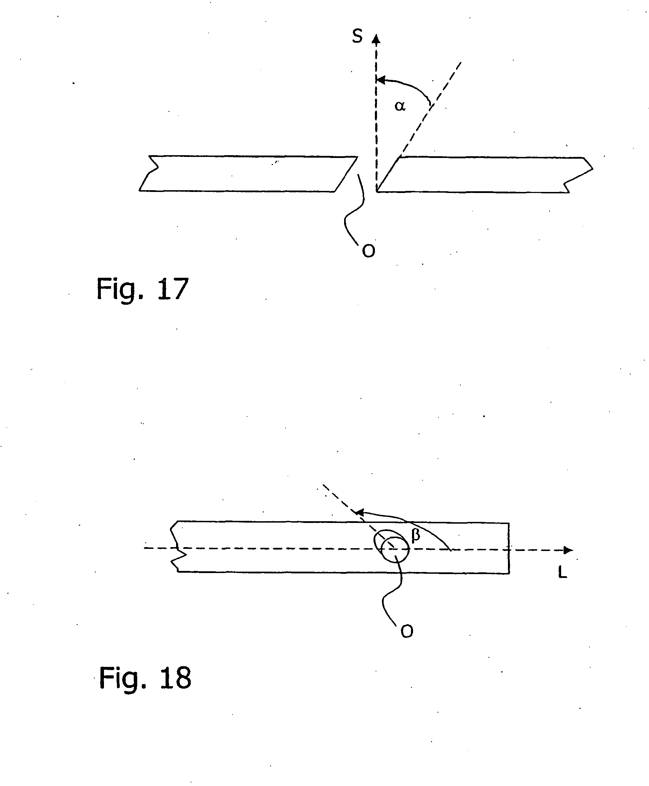

Grundsätzlich lässt sich bei einer linearen Osteosyntheseplatte die Ausrichtung von schräg zur Plattenebene verlaufenden Durchgangsöffnungen durch zwei Winkel α und β eindeutig beschreiben. Dieser Sachverhalt wird nun unter Bezugnahme auf die Fign. 17 und 18 erläutert.

Wie in Fig. 17 dargestellt, bezeichnet ein erster Winkel α die Neigung einer Durchgangsöffnung O bezüglich einer Senkrechten S zur Plattenebene. Die Plattenebene verläuft in Fig. 17 senkrecht zur Zeichenebene. Ein zweiter Winkel β bezeichnet gemäß Fig. 18 eine Winkelausrichtung der Durchgangsöffnung O innerhalb der Plattenebene bezüglich einer Plattenlängsachse L. Die Plattenebene verläuft in Fig. 18 parallel zur Zeichenebene.

Die Winkel α und β gestatten eine eindeutige Winkelcharakterisierung dann, wenn man den ersten Winkel α auf den Bereich von 0 bis 90° beschränkt und den zweiten Winkel β von 0 bis 360° laufen lässt. In den nachfolgenden Ausführungen werden alle Winkel im Gegenuhrzeigersinn und bezüglich einer gerichteten Referenzgeraden (beispielsweise bezüglich einer in eine bestimmte Richtung zeigenden Plattenlängsachse) angegeben.

In der US 5,558,674 ist in den Fign. 5 und 6 eine lineare Osteosyntheseplatte dargestellt, die insgesamt vier schräg zur Plattenebene verlaufende Durchgangsöffnungen aufweist. Jede dieser vier Durchgangsöffnungen schneidet die Plattenebene ungefähr unter einem Neigungswinkel α = 45°. Bezüglich einer auf das freie Ende 21 der Osteosyntheseplatte 20 zeigenden Plattenlängsachse besitzt die schräg verlaufende Durchgangsöffnung 26b eine Winkelausrichtung β = 0°. Die restlichen drei schräg verlaufenden Durchgangsöffnungen besitzen eine entgegengesetzte Winkelausrichtung β = 180°.

Aus der DE 199 62 317 A1 ist eine lineare Osteosyntheseplatte mit zwei senkrecht zur Plattenebene verlaufenden und mit zwei schräg zur Plattenebene verlaufenden Durchgangsöffnungen bekannt. Bei dieser Osteosyntheseplatte weisen die beiden schräg zur Plattenebene verlaufenden Durchgangsöffnungen jeweils ungefähr einen Neigungswinkel α = 65° bezüglich einer zur Plattenebene senkrechten Geraden auf. Die Winkelausrichtung innerhalb der Plattenebene beträgt bei der ersten schräg verlaufenden Durchgangsöffnung β = 0° bezüglich der Plattenlängsachse und bei der zweiten schräg verlaufenden Durchgangsöffnung β = 180°.

Aus Christian Krenkel, Biomechanics and Osteosynthesis of Condylar Neck Fractures of the Mandible, Quintessence Publishing Co, Inc., Carol Stream, Illinois, 1994, Seiten 56 bis 60, sind weitere lineare Osteosyntheseplatten bekannt, die zur Behandlung von Unterkieferfrakturen eingesetzt werden. Da aus ästhetischen Gründen (zur Vermeidung von Narben im Gesichtsbereich) Brüche im Bereich oberhalb des Unterkieferwinkels durch einen chirurgischen Zugang von der Kieferunterseite her behandelt werden sollen, werden die Durchgangsöffnungen der Osteosyntheseplatten schräg zur Plattenebene ausgebildet. Bei den vorgeschlagenen Osteosyntheseplatten liegt der Neigungswinkel a zwischen 30° und 90°. Die Winkelausrichtung β der Durchgangsöffnungen beträgt entweder 0°, 45°, 90° oder 135°.

Die WO 00/66012 A1 beschreibt eine Osteosyntheseplatte gemäβ dem Oberbegriff von Anspruch 1 mit schräg zueinander verlaufenden Plattenabschnitten. Die Plattenabschnitte weisen Langlöcher auf, die senkrecht zur Plattenebene verlaufen.

Die DE 92 08 234 U1 beschreibt eine Osteosyntheseplatte mit Langlöchern, die eine wannenförmig-sphärische Anpassung an einen entsprechenden Schraubenkopf aufweisen.

Der Erfindung liegt die Aufgabe zugrunde, eine Osteosyntheseplatte zur Versorgung von Frakturen, insbesondere von Frakturen im Bereich des Kopfes wie Unterkieferfrakturen, bereitzustellen, die bei verbesserter Funktionalität auf eine einfache Weise am Knochen fixierbar ist.

### Kurzer Abriss der Erfindung

Diese Aufgabe wird gelöst durch eine Osteosyntheseplatte mit einer Plattenebene, einem sich im Wesentlichen innerhalb der Plattenebene erstreckenden linearen ersten Abschnitt mit einer ersten Längsachse, einem sich im Wesentlichen innerhalb der Plattenebene schräg zum ersten Abschnitt erstreckenden linearen zweiten Abschnitt mit einer zweiten Längsachse, wenigstens einer kreisrunden ersten Durchgangsöffnung im ersten Abschnitt, die schräg zur Plattenebene verläuft und bezüglich der ersten Längsachse eine erste Winkelausrichtung innerhalb der Plattenebene aufweist, und wenigstens einer kreisrunden zweiten Durchgangsöffnung im zweiten Abschnitt, die schräg zur Plattenebene verläuft und bezüglich der ersten Längsachse des ersten Abschnitts eine zweite Winkelausrichtung innerhalb der Plattenebene aufweist, wobei die erste und die zweite Winkelausrichtung bezüglich der ersten Längsachse um weniger als ungefähr 60° voneinander abweichen, so dass Befestigungselemente in einer durch einen einzigen intraoralen Zugang vorgegebenen Richtung in die Durchgangsöffnungen beider Plattenabschnitte einführbar sind.

Obwohl sich die Osteosyntheseplatte jedenfalls im Grund- oder Auslieferungszustand im Wesentlichen innerhalb einer allgemeinen Plattenebene erstreckt, schließt dies nicht aus, dass die Platte oder einzelne Abschnitte der Platte vor oder bei Gebrauch aus der Plattenebene heraus gebogen werden. So kann es zweckmäßig sein, die Osteosyntheseplatte vor deren Befestigung an einem Knochen und/oder Knochenfragment durch Biegen an die besonderen anatomischen Gegebenheiten des Frakturbereichs anzupassen. Dieses Anpassen erfolgt in der Regel manuell seitens des behandelnden Chirurgen. Es ist aber auch möglich, dass die Osteosyntheseplatte in Übereinstimmung mit anatomischen Gegebenheiten bereits im Auslieferungszustand etwas aus der allgemeinen Plattenebene heraus gebogen ist. Derartige Osteosyntheseplatten sind vom Schutzbereich der Erfindung mit umfasst.

Die Winkelausrichtungen der ersten Durchgangsöffnung und der zweiten Durchgangsöffnung bezüglich der als Referenzachse dienenden ersten Längsachse können identisch oder unterschiedlich sein. Oftmals sind etwas voneinander abweichende Winkelausrichtungen um mehr als 0° oder mehr als 10° (bis etwa 60° oder bis etwa 45°) bezüglich der ersten Längsachse aus Handhabungsgründen sinnvoll. Außerdem ist es denkbar, dass die erste Winkelausrichtung schräg zur ersten Längsachse und/oder die zweite Winkelausrichtung schräg zur zweiten Längsachse verläuft. Dies bedeutet im Bild der Fig. 18, dass der Winkel β abweichend von 0° und auch abweichend von 180° gewählt wird. So kann der Winkel β zwischen ungefähr 10° und 170° oder zwischen ungefähr 190° und 350° gewählt werden.

Die Winkel der ersten Durchgangsöffnung und der zweiten Durchgangsöffnung schräg zur Plattenebene (also die Neigungswinkel α im Bild der Fig. 17) können gleich oder unterschiedlich gewählt werden. Die erste Durchgangsöffnung kann die Plattenebene unter einen Neigungswinkel von ungefähr 20° bis 80° schneiden. Auch ein Schnittwinkel innerhalb des Bereichs von ungefähr 30° bis 70° ist denkbar. Der Neigungswinkel, unter dem die zweite Durchgangsöffnung die Plattenebene schneidet, kann sich ebenfalls in diesen Winkelbereichen von ungefähr 20° bis 80° oder von ungefähr 30° bis 70° bewegen.

Gemäß einer ersten Variante beträgt die erste Winkelausrichtung zur ersten Längsachse zwischen ungefähr +90° und -90°, zwischen ungefähr +60° und -60° oder zwischen ungefähr +40° und -40° (beispielsweise bezüglich einer dem zweiten Abschnitt abgewandten oder einem freien Ende des ersten Abschnitts zugewandten Richtung). Gemäß einer mit der ersten Variante kombinierbaren zweiten Variante beträgt die zweite Winkelausrichtung zur zweiten Längsachse zwischen ungefähr 60° und 180° oder zwischen ungefähr 70° und 130° (beispielsweise bezüglich einer dem ersten Abschnitt abgewandten oder einem freien Ende des zweiten Abschnitts zugewandten Richtung). Gemäß einer mit der ersten Variante kombinierbaren dritten Variante beträgt die zweite Winkelausrichtung zur zweiten Längsachse zwischen ungefähr 180° und 300° oder zwischen ungefähr 220° und 290° (beispielsweise bezüglich einer dem ersten Abschnitt abgewandten oder einer einem freien Ende des zweiten Abschnitts zugewandten Richtung). Die zweite Variante und die dritte Variante können sich auf Osteosyntheseplatten für unterschiedliche Körperhälften (rechts/links) beziehen.

Der erste Abschnitt und der zweite Abschnitt können unmittelbar aneinander angrenzen oder können durch einen oder mehrere Verbindungsabschnitte miteinander verbunden sein. Die Verbindungsabschnitte können eine lineare oder eine gebogene Form besitzen.

Bei einem schräg zum ersten Abschnitt verlaufenden zweiten Abschnitt kann der Winkel zwischen dem ersten Abschnitt und dem zweiten Abschnitt zwischen ungefähr 90° bis 160° und insbesondere zwischen ungefähr 110° bis 150° betragen. Der erste Abschnitt und der zweite Abschnitt (bzw. deren Längsachsen) können auch parallelversetzt zueinander verlaufen. In diesem Fall ist zwischen dem ersten Abschnitt und dem zweiten Abschnitt wenigstens ein Verbindungsabschnitt vorgesehen. Der wenigstens eine Verbindungsabschnitt kann sich schräg oder senkrecht zum ersten und zweiten Abschnitt erstrecken.

Um einem Chirurgen das bereits erwähnte Anpassen der Osteosyntheseplatte an anatomische Gegebenheiten zu erleichtern, kann die Osteowntheseplatte wenigstens einen Biegebereich von reduzierter Plattenstärke und/oder von reduzierter Plattenbreite und/oder von mäandernder Form aufweisen. Gemäß einer ersten Variante ist der Biegebereich (z.B. als Verbindungsabschnitt) am Übergang zwischen dem ersten Abschnitt und dem zweiten Abschnitt ausgebildet. Gemäß einer mit der ersten Variante kombinierbaren zweiten Variante ist der Biegebereich zwischen zwei benachbarten Durchgangsöffnungen vorgesehen.

Die Osteosyntheseplatte wird in Abhängigkeit von der jeweils vorgesehenen Indikation dimensioniert. Vor allem bei Indikationen im Kieferbereich kann der erste Abschnitt der Osteosyntheseplatte eine Länge zwischen ungefähr 3 und 100 mm (z.B. zwischen 5 und 60 mm und vorzugsweise zwischen 6 und 25 mm) aufweisen und der zweite Abschnitt kann eine Länge zwischen ungefähr 3 und 100 mm (z.B. zwischen 5 und 60 mm und vorzugsweise zwischen 6 und 25 mm) besitzen. Die Gesamtlänge der Platte kann sich zwischen ungefähr 6 und 200 mm bewegen.

Die Osteosyntheseplatte kann im Bereich des ersten Abschnitts und/oder im Bereich des zweiten Abschnitts eine maximale Plattenstärke zwischen ungefähr 0,5 und 3,5 mm aufweisen. Bei einer möglichen Ausgestaltung ist die Plattenstärke derart gewählt, dass ein Kopf eines Befestigungselements (jedenfalls größtenteils) in der Platte versenkbar ist. Um das Versenken des Kopfes zu unterstützen, kann die wenigstens eine erste Durchgangsöffnung und/oder die wenigstens eine zweite Durchgangsöffnung unterhalb einer Plattenoberfläche einen Anschlag für den Kopf des Befestigungselements aufweisen.

Um eine zuverlässige Befestigung der Osteosyntheseplatte zu ermöglichen, kann eine Mehrzahl (beispielsweise wenigstens 2 bis ungefähr 5) erster Durchgangsöffnungen und eine Mehrzahl (beispielsweise wenigstens 2 bis ungefähr 5) zweiter Durchgangsöffnungen vorgesehen werden. Hierbei kann der gegenseitige Abstand der ersten Durchgangsöffnungen verschieden vom gegenseitigen Abstand der zweiten Durchgangsöffnungen sein. Diese Ausgestaltung ist vor allem dann zweckmäßig, wenn die Länge des ersten Abschnitts von der Länge des zweiten Abschnitts abweicht. Die Durchgangsöffnungen können einen Durchmesser von ungefähr 1,5 bis 3,5 mm, vorzugsweise von ungefähr 2 bis 3 mm, besitzen.

### Kurze Beschreibung der Zeichnungen

Weitere Ausgestaltungen und Vorteile der Erfindung ergeben sich aus der nachfolgenden Beschreibung bevorzugter Ausführungsbeispiele sowie aus den Figuren. Es zeigen:
- Fign. 1 und 2: jeweils eine Aufsicht eines ersten Ausführungsbeispiels einer Osteosyntheseplatte;
- Fig. 3: einen Schnitt entlang der Linie A-A in Fig. 1;
- Fig. 4: einen Schnitt entlang der Linie B-B in Fig. 2;
- Fig. 5: einen Schnitt entlang der Linie C-C in Fig. 1;
- Fig. 6: eine Seitenansicht der Osteosyntheseplatte des ersten Ausführungsbeispiels;
- Fig. 7: eine perspektivische Ansicht der Osteosyntheseplatte des ersten Ausführungsbeispiels;
- Fign. 8A und 8B: die Osteosyntheseplatte des ersten Ausführungsbeispiels mit in Durchgangsöffnungen aufgenommenen Knochenschrauben in jeweils einer perspektivischen Ansicht;
- Fig. 9: ein zweites Ausführungsbeispiel einer Osteosyntheseplatte in einer Aufsicht;
- Fig. 10: eine Osteosyntheseplatte in einer perspektivischen Ansicht;
- Fign. 11A und 11B: ein weiteres Ausführungsbeispiel einer Osteosyntheseplatte mit in Durchgangsöffnungen aufgenommenen Knochenschrauben in jeweils einer perspektivischen Ansicht;
- Fign. 12A und 12B: eine Osteosyntheseplatte mit in Durchgangsöffnungen aufgenommenen Knochenschrauben in jeweils einer perspektivischen Ansicht;
- Fign. 13A und 13B: eine weitere Osteosyntheseplatte mit in Durchgangsöffnungen aufgenommenen Knochenschrauben in jeweils einer perspektivischen Ansicht;
- Fign. 14A und 14B: eine weitere Osteosyntheseplatte insbesondere zur Versorgung von Kieferfrakturen in zwei perspektivischen Ansichten;
- Fign. 15A und 15B: eine weitere Osteosyntheseplatte insbesondere zur Versorgung von Kieferfrakturen in linearem Grundzustand und gebogenem Anwendungszustand;
- Fig. 16: eine weitere Osteosyntheseplatte insbesondere zur Versorgung von Kieferfrakturen in linearem Grundzustand;
- Fig. 17: eine schematische Erläuterung des Neigungswinkels α zwischen einer schräg zur Plattenebene verlaufenden Durchgangsöffnung und einer Senkrechten zur Plattenebene; und
- Fig. 18: eine schematische Erläuterung der Winkelausrichtung β innerhalb der Plattenebene für eine schräg zur Plattenebene verlaufende Durchgangsöffnung.

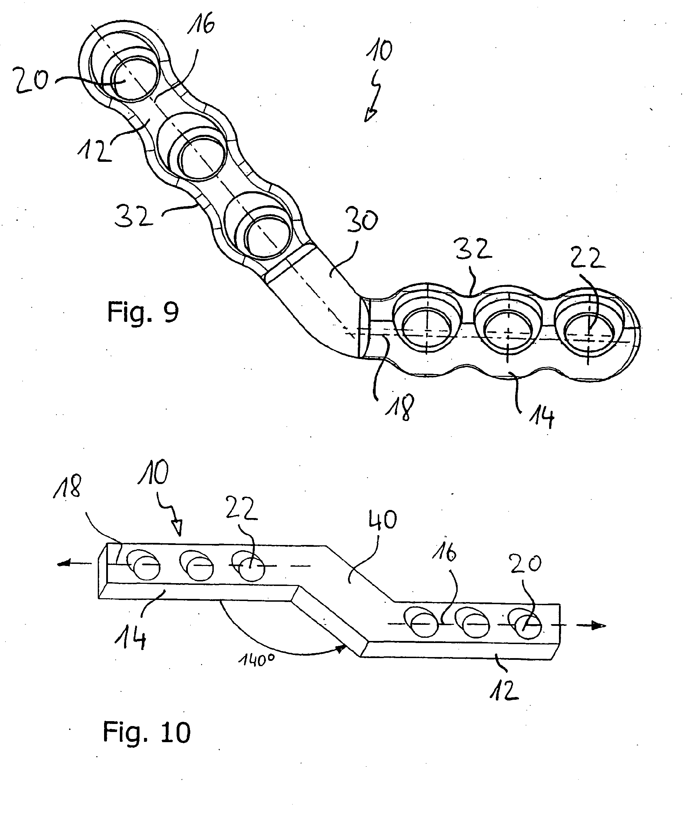

Die Figuren 10 und 12A bis 16 stellen nicht die Erfindung gemäβ Anspruch 1 das, da der erste und der zweite Abschnitt nicht innerhalb der Plattenebene schräg zueinander verlaufen.

### Beschreibung bevorzugter Ausführungsbeispiele

Im Folgenden wird die erfindungsgemäße Osteosyntheseplatte anhand mehrerer Ausführungsbeispiele erläutert. Übereinstimmende Elemente werden hierbei mit denselben Bezugszeichen bezeichnet.

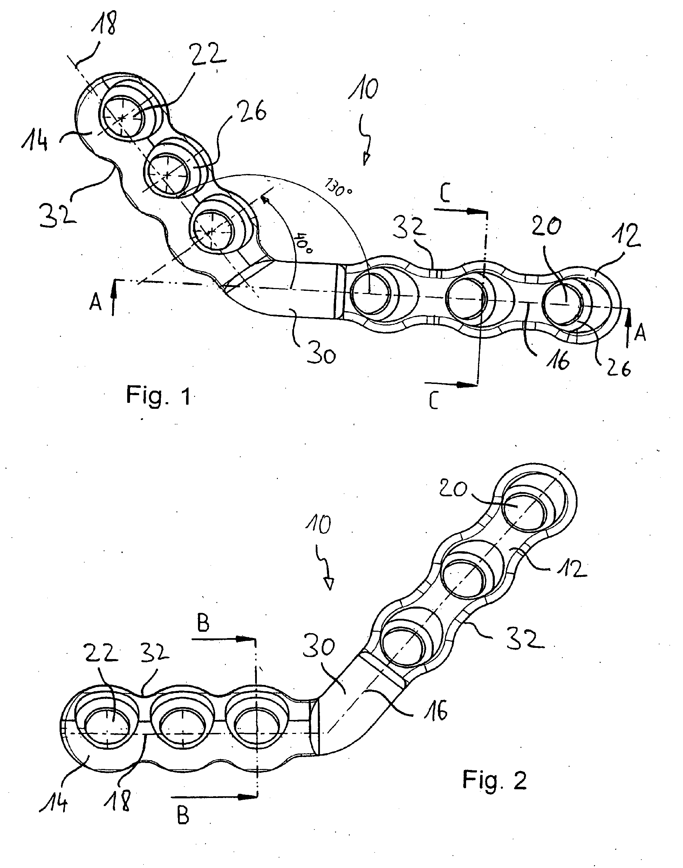

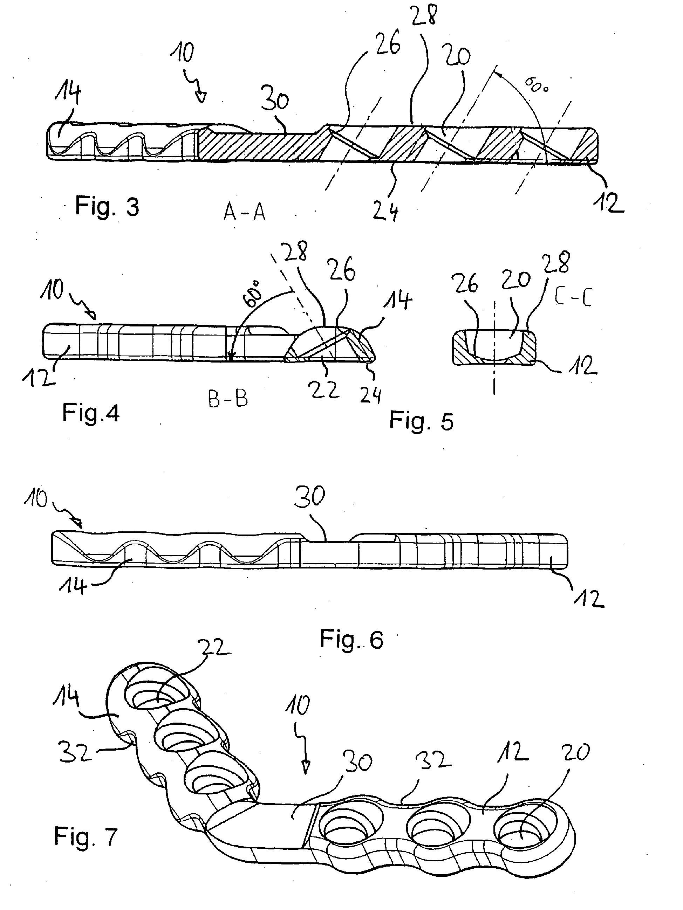

Die Fign. 1 und 2 zeigen jeweils eine Aufsicht eines ersten Ausführungsbeispiels einer Osteosyntheseplatte 10 in unterschiedlichen Ausrichtungen. Die Fign. 3 bis 7 und die Fign. 8A und 8B zeigen weitere Ansichten dieser Osteosyntheseplatte 10.

Die Osteosyntheseplatte 10 besteht aus Titan und ist insbesondere zur Versorgung von Kieferfrakturen (insbesondere von Frakturen im Bereich des Unterkieferwinkels) geeignet. Bei der in den Fign. 1 bis 7, 8A und 8B dargestellten Osteosyntheseplatte 10 handelt es sich um eine Platte für den rechten Unterkieferwinkel. Für den linken Kieferwinkel ist die in Fig. 9 dargestellte Platte vorgesehen. Bei der linken Osteosyntheseplatte der Fig. 9 handelt es sich um das spiegelsymmetrische Gegenstück zur rechten Osteosyntheseplatte 10. Aus diesem Grund gelten die Ausführungen zur rechten Osteosyntheseplatte 10 bis auf wenige Ausnahme auch für die linke Osteosyntheseplatte gemäß Fig. 9. Auf die Ausnahmen wird im Zusammenhang mit der Erläuterung der Fig. 9 näher eingegangen.

Die Osteosyntheseplatte 10 gemäß dem ersten Ausführungsbeispiel erstreckt sich im Auslieferungszustand innerhalb einer allgemeinen Plattenebene, die in den Fign. 1 und 2 parallel zur Zeichenebene verläuft. Die Osteosyntheseplatte 10 besitzt zwei aneinander angrenzende lineare Plattenabschnitte 12, 14 mit zugehörigen Längsachsen 16, 18. Die beiden Plattenabschnitte 12, 14 verlaufen innerhalb der Plattenebene schräg zueinander. Wie Fig. 1 entnommen werden kann, beträgt der Schnittwinkel zwischen den Längsachsen 14, 16 der beiden Plattenabschnitte 12, 14 im Ausführungsbeispiel ungefähr 130°. Die Länge des Plattenabschnitts 12 (gemessen vom Schnittpunkt der beiden Längsachsen 16, 18 bis zum freien Ende des Abschnitts 12) beträgt ungefähr 14 mm und die Länge des Plattenabschnitts 14 (gemessen vom Schnittpunkt der beiden Längsachsen 16, 18 bis zum freien Ende des Abschnitts 14) beträgt ungefähr 10 mm.

Im Plattenabschnitt 12 sind drei gleichartige Durchgangsöffnungen 20 und im Plattenabschnitt 14 drei ebenfalls gleichartige Durchgangsöffnungen 22 ausgebildet. Die Durchgangsöffnungen 20, 22 besitzen im engsten Bereich einen Durchmesser von 2,4 mm.

Die Durchgangsöffnungen 20 im Plattenabschnitt 12 schneiden die Plattenebene unter einen Neigungswinkel von α = 60°. Dieser Sachverhalt ergibt sich aus Fig. 3, die einen Schnitt entlang der Linie A-A von Fig. 1 zeigt. Die Durchgangsöffnungen 22 des Plattenabschnitts 14 schneiden die Plattenebene ebenfalls unter einem Neigungswinkel von α = 60°. Dies kann Fig. 4 entnommen werden, welche einen Schnitt entlang der Linie B-B von Fig. 2 zeigt. Was die Definition des Winkels α anbelangt, wird auf Fig. 17 Bezug genommen.

Die Durchgangsöffnungen 20 des Plattenabschnitts 12 weisen innerhalb der Plattenebene bezüglich der Längsachse 16 eine Winkelausrichtung von β = 0° auf. Die Winkelausrichtung bezüglich der Längsachse 16 wird in Richtung auf ein freies Ende des Plattenabschnitts 12 bestimmt. Die Durchgangsöffnungen 22 des Plattenabschnitts 14 weisen innerhalb der Plattenebene bezüglich der Längsachse 18 (und in Richtung auf das freie Ende des Plattenabschnitts 14) eine Winkelausrichtung von β = 270° auf. Bezüglich der Längsachse 16 des Plattenabschnitts 12 besitzen die Durchgangsöffnungen 22 eine Winkelausrichtung β = 40° (wiederum in Richtung auf das freie Ende des Plattenabschnitts 12 bezogen). Die Winkelausrichtung von β = 40° der Durchgangsöffnungen 22 des Plattenabschnitts 14 bezüglich der Längsachse 16 des Plattenabschnitts 12 ist in Fig. 1 eingezeichnet. Was die Bestimmung des Winkels β anbelangt, wird auf Fig. 18 Bezug genommen.

Bei der Osteosyntheseplatte 10 gemäß den Fign. 1 bis 7, 8A und 8B besitzen folglich die Durchgangsöffnungen 20 eine Winkelausrichtung in der Plattenebene von β = 0° und die Durchgangsöffnungen 22 eine Winkelausrichtung in der Plattenebene von β = 40° (jeweils bezogen auf die Längsachse 16 des Plattenabschnitts 12). Die Differenz der Winkelausrichtungen der Durchgangsöffnungen 20 und der Durchgangsöffnungen 22 innerhalb der Plattenebene beträgt daher ungefähr 40°.

Wie vor allem in den Fign. 3 und 4 gut erkennbar, besitzen die Durchgangsöffnungen 20 im Plattenabschnitt 12 (genauso wie die Durchgangsöffnungen 22 im Plattenabschnitt 14) einen sich in Richtung auf die Plattenunterseite 24 stufenartig verringernden Innendurchmesser. Auf diese Weise entsteht innerhalb der Durchgangsöffnungen 20, 22 jeweils eine als Anschlag fungierende Anlagefläche 26 für den Kopf eines Befestigungselements. Die Anlagefläche 26 ist unterhalb der Plattenoberfläche 28 und oberhalb der Plattenunterseite 24 ausgebildet. Da jedenfalls der tiefste Bereich der Anlagefläche 26 (vgl. Fig. 5) unterhalb der Plattenoberfläche 28 liegt, lässt sich der Kopf eines in die Durchgangsöffnungen 20, 22 eingeführten Befestigungselements zumindest teilweise in der Osteosyntheseplatte 10 versenken.

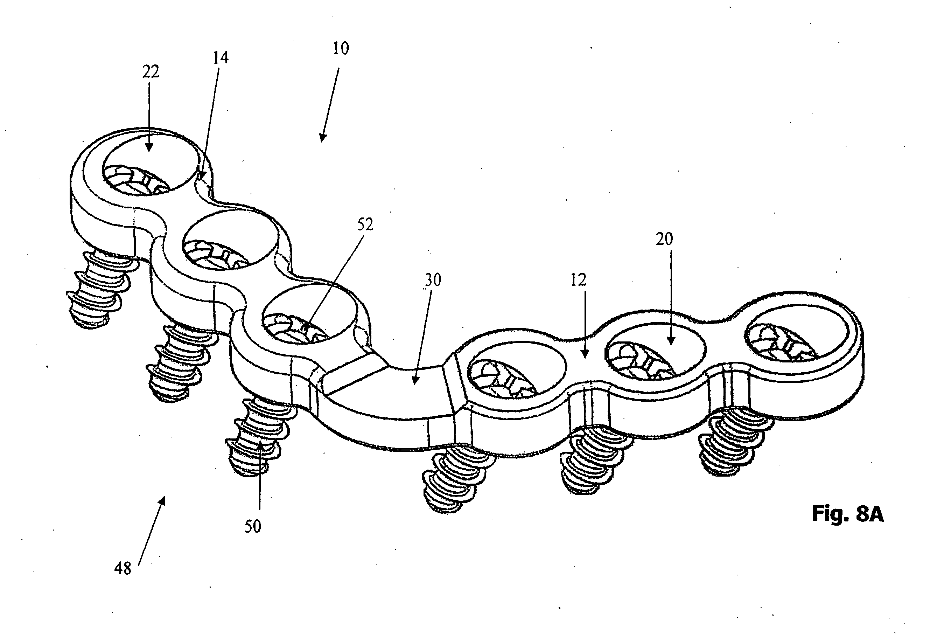

In der Fig. 8A ist deutlich zu erkennen, dass die Schäfte 50 von Knochenschrauben 48 im Plattenabschnitt 12 bis auf die unterschiedliche Winkelausrichtung (Δβ = 40°) im Wesentlichen parallel zu den Schäften 50 von Knochenschrauben 48 im Plattenabschnitt 14 verlaufen. Ferner ist in der Darstellung gemäß Fig. 8A gut zu erkennen, dass die Köpfe 52 der Knochenschrauben 48 bezüglich der Plattenoberseite versenkt aufgenommen werden.

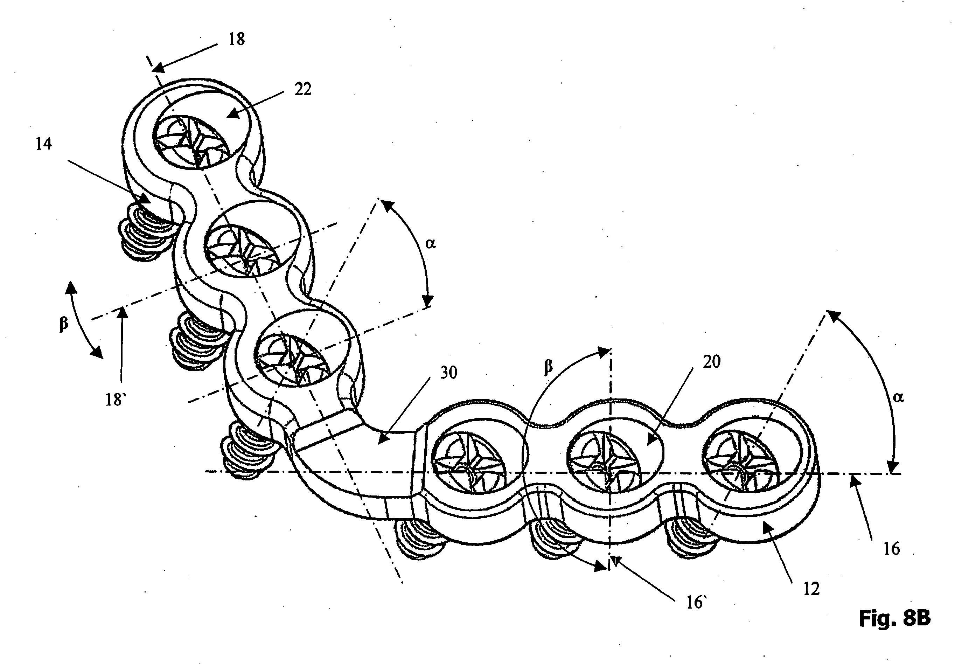

Hinsichtlich der Fig. 8B sei noch erwähnt, dass die dort eingezeichneten, senkrecht zu den Längsachsen 16, 18 verlaufenden Hilfslinien 16', 18' zur Verdeutlichung des Winkelausrichtungsbereichs β dienen. Wie in Fig. 8B eingezeichnet, kann die Winkelausrichtung β bezüglich der Hilfslinien 16', 18' um ±90°, vorzugsweise um ungefähr ±60°, variieren.

Die im Auslieferungszustand planare Osteosyntheseplatte 10 besitzt mehrere Biegebereiche von reduzierter Plattenstärke oder reduzierter Plattenbreite. Diese Biegebereiche ermöglichen es dem Chirurgen, die Osteosyntheseplatte 10 an die anatomischen Gegebenheiten im Frakturbereich anzupassen. Dabei kann die Osteosyntheseplatte 10 mittels geeigneter Werkzeuge wie Biegezangen sowohl innerhalb der Plattenebene als auch aus der Plattenebene heraus gebogen werden.

Ein erster Biegebereich 30 der Osteosyntheseplatte 10 ist gemäß Fig. 1 am Übergang zwischen dem Plattenabschnitt 12 und dem Plattenabschnitt 14 angeordnet. Wie sich aus der Seitenansicht gemäß Fig. 6 ergibt, weist die Osteosyntheseplatte 10 im Biegebereich 30 eine minimale Breite und eine geringere Höhe als in Bereichen außerhalb des Biegebereichs 30 auf. Diese stellenweise Reduzierung der Plattenstärke (von maximal ca. 2 mm außerhalb des Biegebereichs 30 auf ca. 1,5 mm im Biegebereich 30) und der Plattenbreite erleichtert dem Chirurgen das Biegen der Osteosyntheseplatte 10.

Eine Mehrzahl zweiter Biegebereiche 32 sind gemäß Fig. 1 jeweils zwischen zwei benachbarten Durchgangsöffnungen 20 des Plattenabschnitts 12 und auch zwischen zwei benachbarten Durchgangsöffnungen 22 des Plattenabschnitts 14 ausgebildet. Diese weiteren Biegebereiche 32 werden von Bereichen verringerter Plattenbreite gebildet.

Fig. 9 zeigt die linke Osteosyntheseplatte 10 eines Plattensystems, welches auch die oben unter Bezugnahme auf die Fign. 1 bis 7, 8A und 8B erläuterte rechte Osteosyntheseplatte umfasst. Wie bereits erwähnt, handelt es sich bei der linken Osteosyntheseplatte 10 um das spiegelsymmetrische Gegenstück zur rechten Osteosyntheseplatte. Demgemäss besteht der maßgebliche Unterschied zur rechten Osteosyntheseplatte darin, dass die Durchgangsöffnungen 22 des Plattenabschnitts 14 eine andere Winkelausrichtung innerhalb der Plattenebene aufweisen. Während bei der rechten Platte die entsprechende Winkelausrichtung β = 270° beträgt, weisen die Durchgangsöffnungen 22 der linken Osteosyntheseplatte 10 bezüglich der Längsachse 18 und in Richtung auf das freie Ende des Plattenabschnitts 14 eine spiegelbildliche Spiegelausrichtung β = 90° auf. Die Differenz der Winkelausrichtungen der Durchgangsöffnungen 22 des Plattenabschnitts 14 und der Durchgangsöffnungen 20 des Plattenabschnitts 12 (jeweils bezogen auf die Längsachse 16) beläuft sich unverändert auf 40°.

Fig. 10 zeigt eine Osteosyntheseplatte 10 zur Behandlung von Frakturen im Kieferbereich. Die Osteosyntheseplatte 10 besitzt zwei Plattenabschnitt 12, 14, die parallelversetzt zueinander angeordnet sind. Zwischen den beiden Plattenabschnitten 12, 14 ist ein schräg zu jedem dieser Abschnitte 12, 14 verlaufender Verbindungsabschnitt 40 vorgesehen. Der Verbindungsabschnitt 40 schneidet die beiden Plattenabschnitte 12, 14 unter einem Winkel von jeweils ungefähr 140°.

In jedem der beiden Plattenabschnitte 12, 14 sind jeweils drei gleichartige Durchgangsöffnungen 20, 22 ausgebildet. Die Durchgangsöffnungen 20, 22 schneiden die Plattenebene unter einem Neigungswinkel α = 45°. Bezüglich der Längsachse 16 des Plattenabschnitts 12 und in Richtung auf das freie Ende des Plattenabschnitts 12 beträgt die Winkelausrichtung der Durchgangsöffnungen 20 innerhalb der Plattenebene β = 135°. Die Winkelausrichtung der Durchgangsöffnungen 22 bezüglich der Längsachse 18 des Plattenabschnitts 14 und in Richtung auf das freie Ende des Plattenabschnitts 14 beträgt β = 45°. Bezogen auf die Längsachse 16 des Plattenabschnitts 12 und das freie Ende des Plattenabschnitts 12 beträgt die Winkelausrichtung der Durchgangsöffnungen 22 des Plattenabschnitts 14 β = 135°. Die Winkelausrichtungen der Durchgangsöffnungen 20 und der Durchgangsöffnungen 22 stimmen daher bezüglich der Längsachse 16 des Plattenabschnitts 12 überein.

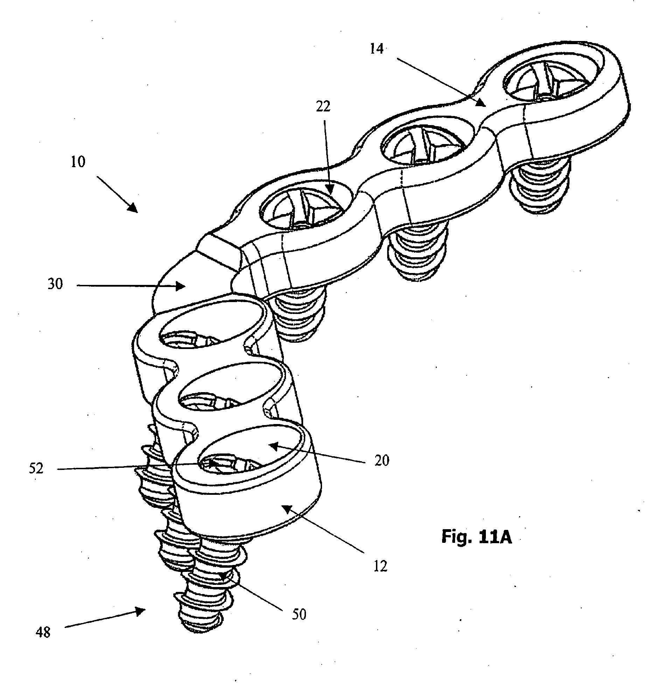

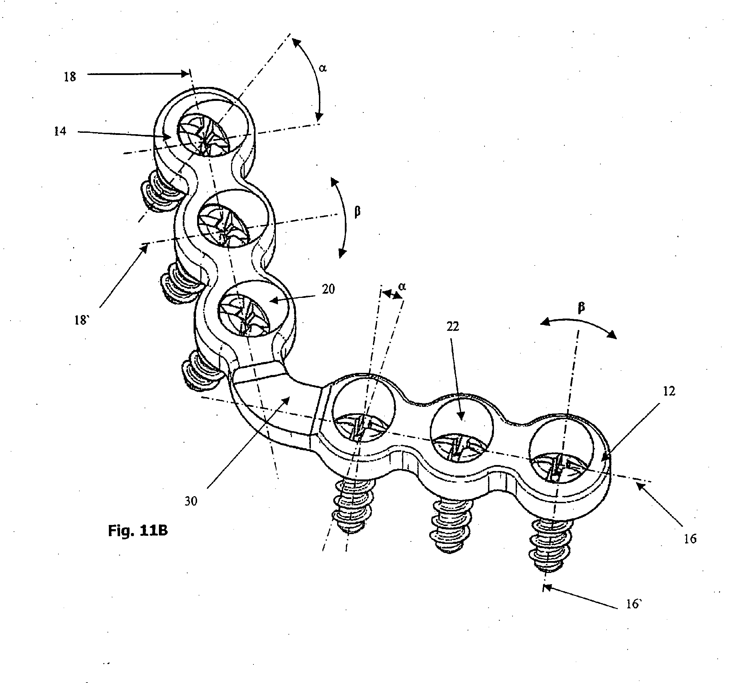

In den Fign. 11A und 11B ist ein weiteres Ausführungsbeispiel einer Osteosyntheseplatte 10 für die Versorgung von Unterkieferwinkelfrakturen dargestellt. Die dargestellte Osteosyntheseplatte 10 stimmt bis auf die Winkelausrichtungen der Durchgangsöffnungen im Wesentlichen mit der unter Bezugnahme auf die Fign. 1 bis 7, 8A und 8B erläuterten Osteosyntheseplatte überein. Aus diesem Grund werden im Folgenden lediglich die Unterschiede erörtert.

In den Fign. 11A und 11B ist ein weiteres Ausführungsbeispiel einer Osteosyntheseplatte 10 dargestellt. Bei diesem Ausführungsbeispiel schließen die beiden Plattenabschnitte 12, 14 wiederum einen Winkel von 130° miteinander ein. Die Durchgangsöffnungen 20, 22 weisen jeweils einen Neigungswinkel von α = 60° bezüglich der Plattenebene auf. Die Winkelausrichtung der Durchgangsöffnungen 20 des Plattenabschnitts 12 innerhalb der Plattenebene (und bezogen auf das freie Ende des Plattenabschnitts 12) beträgt in diesem Ausführungsbeispiel 90°. Wie im ersten Ausführungsbeispiel weisen die Durchgangsöffnungen 22 des Plattenabschnitts 14 innerhalb der Plattenebene bezüglich der Längsachse 18 (und in Richtung auf das freie Ende des Plattenabschnitts 14) eine Winkelausrichtung von β = 270° auf. Die Differenz der Winkelausrichtungen der Durchgangsöffnungen 20 und der Durchgangsöffnungen 22 innerhalb der Plattenebene beträgt ungefähr 50°. Die Winkelausrichtungen der Durchgangsöffnungen 20 und 22 können um ±90°, vorzugsweise um ungefähr ±60°, um die angegebenen Winkelausrichtungen variieren.

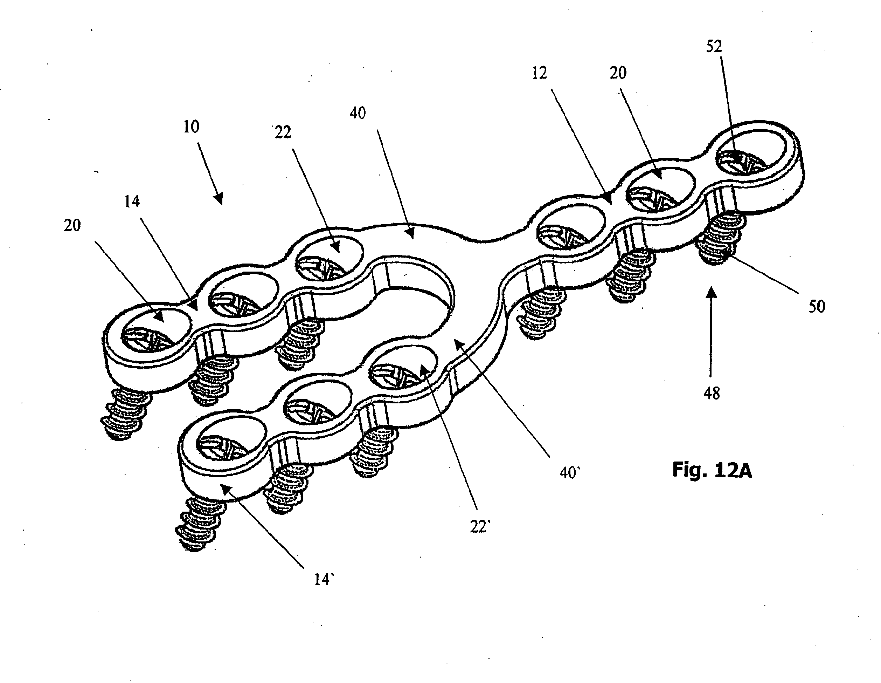

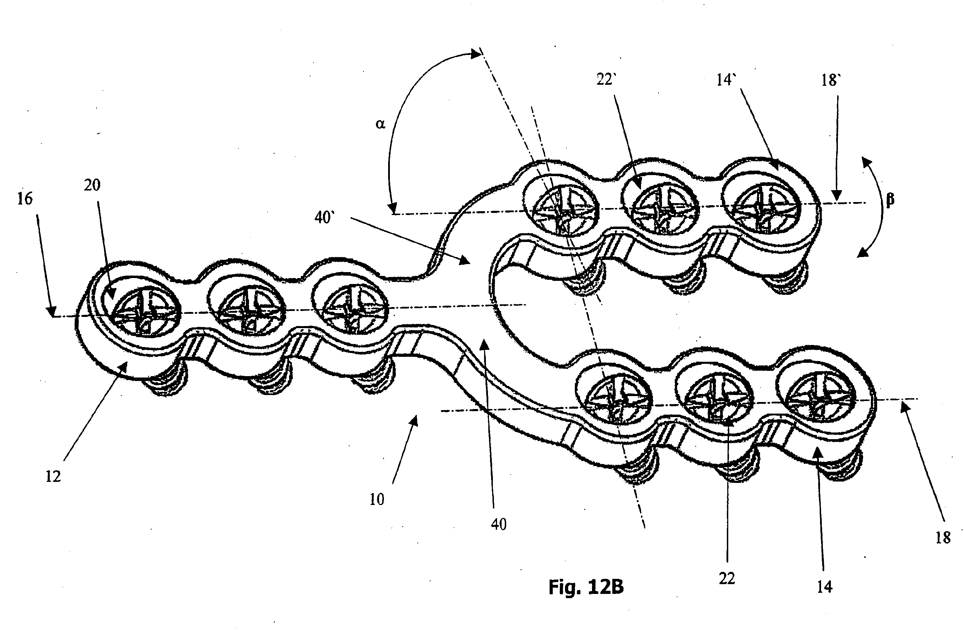

In den Fign. 12A und 12B ist eine Osteosyntheseplatte 10 mit insgesamt drei Plattenabschnitten 12, 14, 14' und einer Gesamtlänge von ungefähr 40 mm abgebildet. Die Osteosyntheseplatte 10 besitzt eine im Wesentlichen gabelförmige Gestalt. Die beiden Plattenabschnitte 14, 14' verlaufen parallelversetzt bezüglich der Längsachse 16 des Abschnitts 12. Der Plattenabschnitt 12 ist über einen jeweils nach Art eines Viertelkreisbogens gebogenen Verbindungsabschnitt 40, 40' mit den Plattenabschnitten 14, 14' verbunden.

Die gabelförmige Gestalt der Osteosyntheseplatte 10 ist dadurch bedingt, dass die beiden Plattenabschnitte 14, 14' einen Nerv (z.B. im Bereich des Unterkiefers) zwischen sich aufnehmen. Auf diese Weise kann eine Verletzung des Nervs durch die Knochenschrauben 48 vermieden werden.

Die Durchgangsöffnungen 20 des Plattenabschnitts 12 der Osteosyntheseplatte 10 sowie die Durchgangsöffnungen 20, 20' der Plattenabschnitte 14, 14' schneiden die Plattenebene jeweils unter einem Neigungswinkel von α = 60°. Die Durchgangsöffnungen 20 des Plattenabschnitts 12 weisen innerhalb der Plattenebene bezüglich der Längsachse 16 und in Richtung auf ein freies Ende des Plattenabschnitts 12 eine Winkelausrichtung von β = 0° auf. Die Durchgangsöffnungen 22, 22' der Plattenabschnitte 14, 14' weisen innerhalb der Plattenebene bezüglich der jeweiligen Längsachse 18, 18' (und in Richtung auf das jeweils freie Ende der Plattenabschnitt 14, 14') eine Winkelausrichtung von β = 180° auf. Bezüglich der Längsachse 16 des Plattenabschnitts 12 besitzen die Durchgangsöffnungen 22, 22' eine Winkelausrichtung β = 0° (wiederum in Richtung auf das freie Ende des Plattenabschnitts 12 bezogen). Die Differenz der Winkelausrichtungen der Durchgangsöffnungen 20 und der Durchgangsöffnungen 22, 22' innerhalb der Plattenebene beträgt daher 0°.

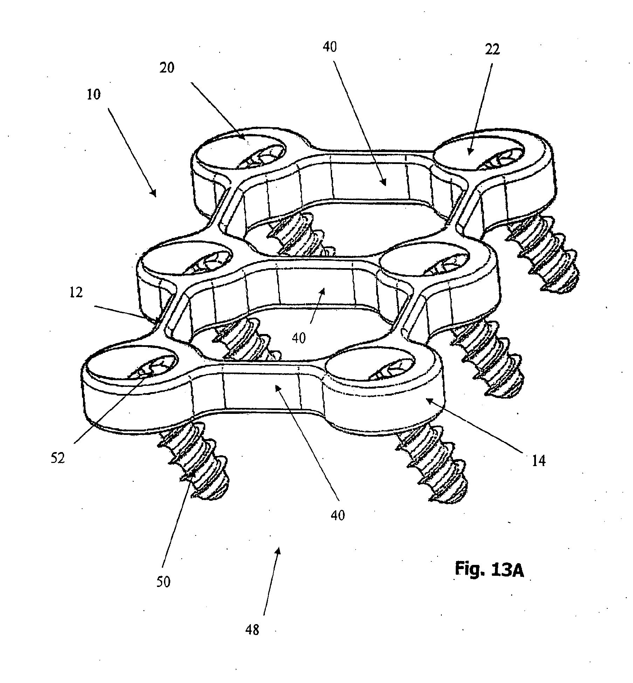

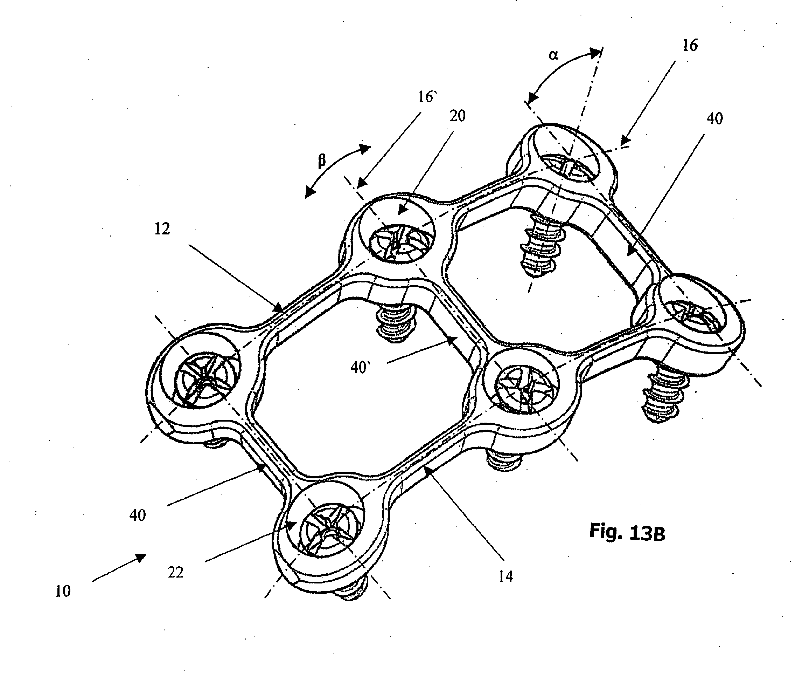

Eine weitere Osteosyntheseplatte 10 ist in den Fign. 13A und 13B abgebildet. Die dort abgebildete Osteosyntheseplatte 10 besitzt eine im Wesentliche gitterförmige Gestalt mit zwei parallelversetzt zueinander verlaufenden Plattenabschnitten 12, 14. Die Plattenabschnitte 12, 14 sind im Bereich gegenüberliegender Durchgangsöffnungen 20, 22 durch jeweils einen Verbindungsabschnitt 40 miteinander verbunden. In dem in den Fign. 13A und 13B dargestellten Beispielfall mit zwei mal drei Durchgangsöffnungen 20, 22 (also drei pro Plattenabschnitt 12, 14) sind demnach drei Verbindungsabschnitte 40 vorgesehen. Die Verbindungsabschnitte 40 verlaufen parallel zueinander und schneiden die Plattenabschnitt 12, 14 im Beispielfall unter einem rechten Winkel. Eine Abwandlung der in den Fign. 13A und 13B dargestellten Osteosyntheseplatte 10 könnte anstatt zwei mal drei Durchgangsöffnungen zwei mal vier oder drei mal vier Durchgangsöffnungen aufweisen.

Die Durchgangsöffnungen 20, 22 der Osteosyntheseplatte 10 der Fign. 13A und 13B schneiden die Plattenebene jeweils unter einem Neigungswinkel von α = 60°. Die Durchgangsöffnungen 20 des Plattenabschnitts 12 weisen innerhalb der Plattenebene bezüglich einer Längsachse des Plattenabschnitts 12 eine Winkelausrichtung von β = 90°/270° (im Beispielfall der Fign. 13A und 13B existiert keine Vorzugsrichtung) auf. Die Durchgangsöffnungen 22 des Plattenabschnitts 14 besitzen bezüglich einer Längsachse des Plattenabschnitts 14 dieselbe Winkelausrichtung von β = 90°/270°. Demgemäss beträgt die Differenz der Winkelausrichtungen der Durchgangsöffnungen 20 und der Durchgangsöffnungen 22 innerhalb der Plattenebene 0°.

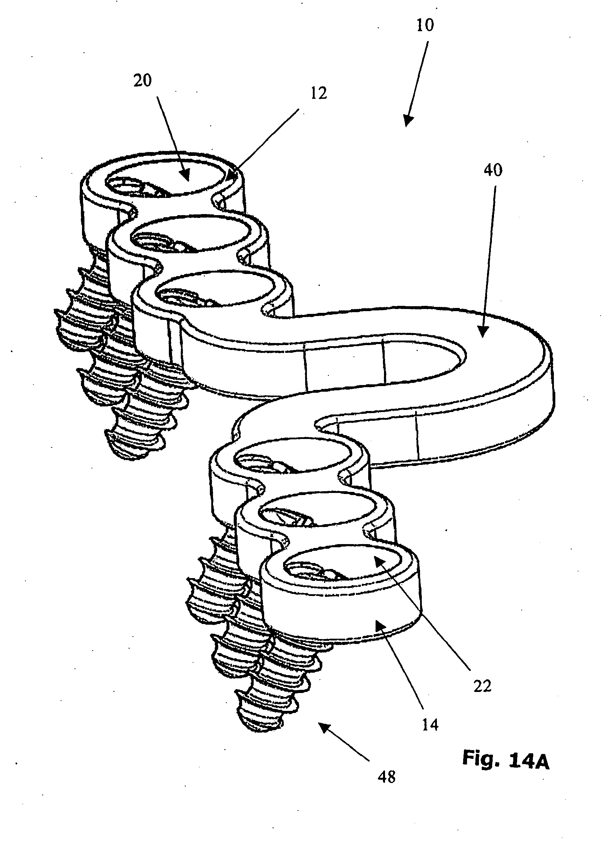

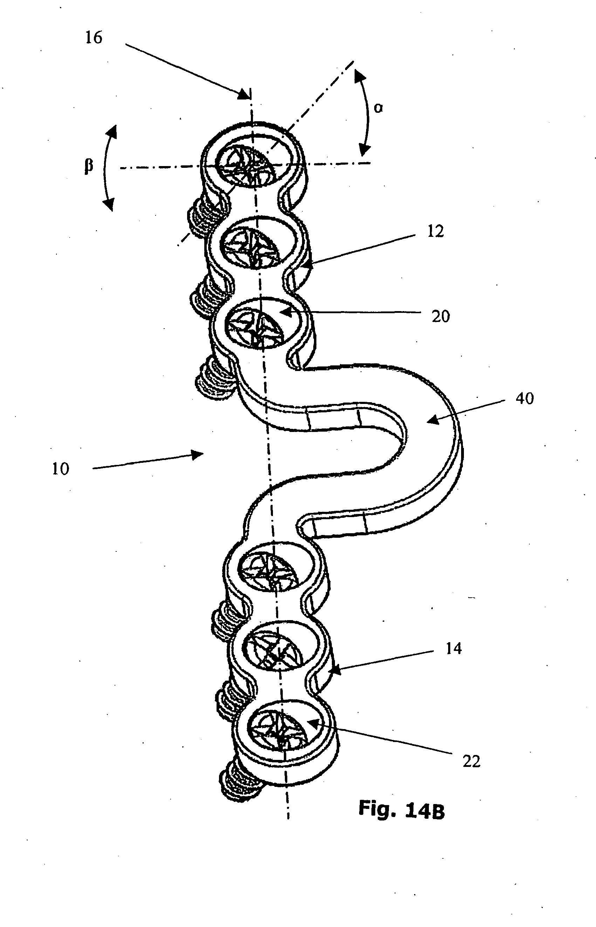

In den Fign. 14A und 14B ist eine weitere Osteosyntheseplatte 10 mit zwei Plattenabschnitten 12, 14 dargestellt. Die beiden Plattenabschnitte 12, 14 besitzen eine gemeinsame Längsachse 16 und sind über einen mäandernd (U-förmig) gebogenen Verbindungsabschnitt 40 miteinander verbunden. Die Osteosyntheseplatte 10 kann im Anwendungszustand derart positioniert werden, dass sich der U-förmig gebogene Verbindungsabschnitt 40 um einen Nerv herum erstreckt. Bei einer Abwandlung der Osteosyntheseplatte 10 gemäß den Fign. 14A und 14B ist im Bereich des Verbindungsabschnitts 40 wenigstens eine Knochenschrauben-Durchgangsöffnung vorgesehen.

Die Durchgangsöffnungen 20, 22 schneiden die Plattenebene jeweils unter einem Neigungswinkel von α = 60°. Die Durchgangsöffnungen 20 des Plattenabschnitts 12 weisen innerhalb der Plattenebene bezüglich der gemeinsamen Längsachse 16 (und in Richtung auf das freie Ende des Plattenabschnitts 12) eine Winkelausrichtung von β = 90° auf. Die Durchgangsöffnungen 22 des Plattenabschnitts 14 besitzen bezüglich der gemeinsamen Längsachse 16 und bezüglich des freien Endes des Plattenabschnitts 14 eine Winkelausrichtung von β = 270°. Die Differenz der Winkelausrichtungen der Durchgangsöffnung 20 und der Durchgangsöffnungen 22 innerhalb der Plattenebene beträgt folglich 0°.

Die Fign. 14A und 14B zeigen die Osteosyntheseplatte 10 im Grundzustand. Gemäß einem weiteren Ausführungsbeispiel der Erfindung lässt sich die Osteosyntheseplatte 10 im Bereich des Verbindungsabschnitts 40 (der dann als Biegebereich fungiert) derart verformen, dass der Plattenabschnitt 12 schräg zum Plattenabschnitt 14 verläuft.

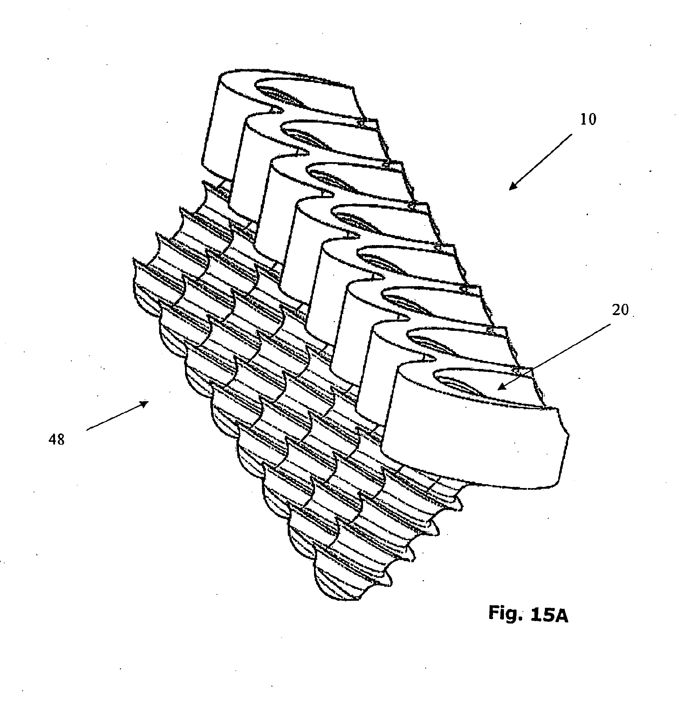

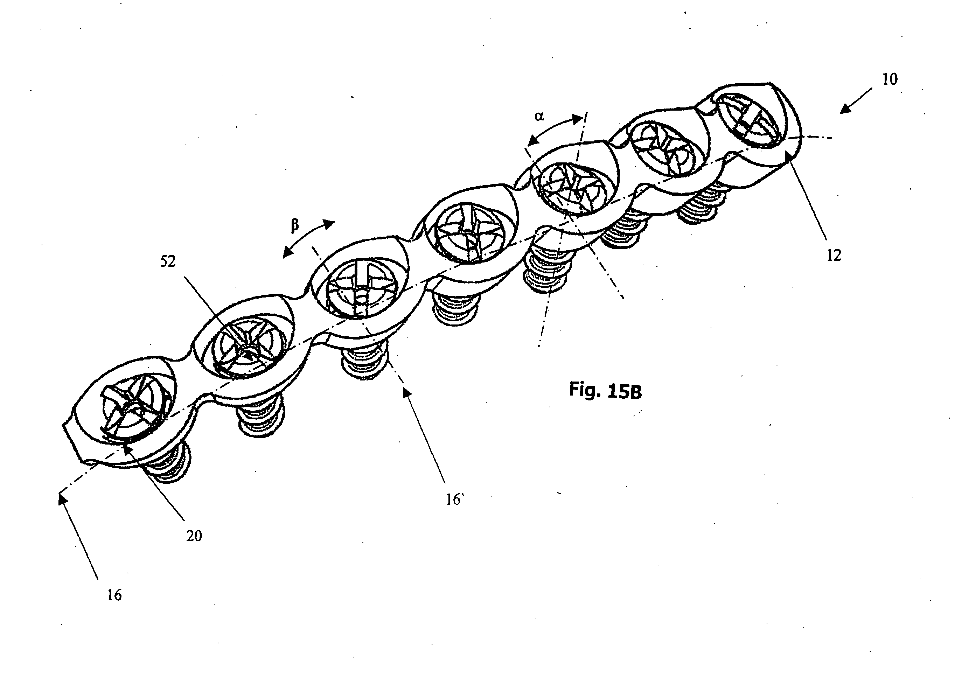

Eine weitere Osteosyntheseplatte 10 ist in den Fign. 15A und 15B dargestellt. Die Osteosyntheseplatte 10 besitzt in dem in Fig. 15A dargestellten Grundzustand eine lineare Gestalt mit insgesamt acht Durchgangsöffnungen 20. Die Durchgangsöffnungen 20 schneiden die Plattenebene unter einem Winkel unter α = 60° und besitzen innerhalb der Plattenebene eine Winkelausrichtung von β = 90°/270° (es existiert keine Vorzugsrichtung). Die Winkelausrichtung β kann bezüglich der in Fig. 15B eingezeichneten Hilfslinie 16' um ±90°, vorzugsweise um ungefähr ±60°, variieren.

Fig. 15B zeigt die Osteosyntheseplatte 10 im gebogenen Anwendungszustand. Die Osteosyntheseplatte 10 ist in diesem Beispiel im Bereich der Vorderseite des Unterkieferknochens (also im Kinnbereich) befestigt und mit ihrer gebogenen Form an die Kontur dieses Knochens angepasst. Da die Durchgangsöffnungen 20, 22 schräg nach oben zeigen, konnten die Schrauben 48 intraoral (und zwar schräg von oben) eingebracht werden.

Die Osteosyntheseplatte gemäß den Fign. 15A, 15B lässt sich innerhalb der Plattenebene ähnlich wie in Fig. 10 dargestellt derart verformen, dass zwei parallelversetzt zueinander verlaufende, lineare Plattenabschnitte entstehen.

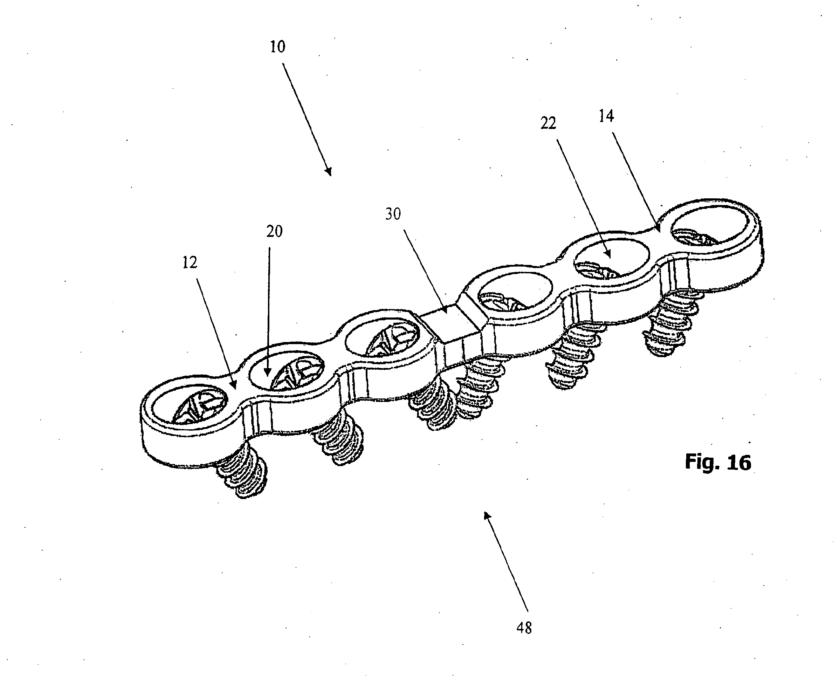

In Fig. 16 ist eine weitere Osteosyntheseplatte 10 dargestellt. Die Osteosyntheseplatte 10 besitzt eine lineare Gestalt und weist zwei über einen als Biegebereich 30 ausgebildeten Verbindungsabschnitt miteinander verbundene Plattenabschnitte 12, 14 auf. Die Durchgangsöffnungen 20, 22 der Plattenabschnitte 12, 14 besitzen einen Neigungswinkel α = 60° bezüglich der Plattenebene. Die Winkelausrichtungen der Durchgangsöffnungen 20, 22 betragen jeweils β = 180° bezüglich der freien Enden des jeweiligen Plattenabschnitts 12, 14. Die Differenz der Winkelausrichtungen der Durchgangsöffnungen 20 und der Durchgangsöffnungen 22 beträgt demgemäss 180°.

Gemäß einem Ausführungsbeispiel der Erfindung wird die in Fig. 16 dargestellte Osteosyntheseplatte 10 an der Stelle des Biegebereichs 30 derart gebogen, dass die beiden Plattenabschnitte 12, 14 schräg und im Wesentlichen V-förmig zueinander verlaufen.

Die unter Bezugnahme auf die Fign. 1 bis 11B erläuterten Osteosyntheseplatten eignen sich für die intraorale Versorgung von Unterkieferwinkelfrakturen. Die unter Bezugnahme auf die Fign. 12A bis 16 beschriebenen Osteosyntheseplatten eignen sich zur intraoralen Versorgung von Kieferfrakturen in vom Unterkieferwinkel beabstandeten Kieferbereichen, z. B. im Bereich des Kinns oder des Condylus.

Das Vorsehen von zwei oder mehr Plattenabschnitten, die nichtlinear zueinander verlaufen, ermöglicht die Versorgung selbst komplizierter Kieferfrakturen mittels einer einzigen Osteosyntheseplatte. Die Ausrichtung der einzelnen Durchgangsöffnungen in der Plattenebene und senkrecht hierzu ist in den Ausführungsbeispielen derart gewählt, dass die Osteosyntheseplatten durch einen intraoralen chirurgischen Eingriff, also durch den Mund hindurch, befestigt werden können. Es ist daher kein transbuccaler Zugang (also durch die Wange hindurch) erforderlich, um die Osteosyntheseplatten der Ausführungsbeispiele zu platzieren und mittels geeigneter Befestigungselemente wie monokortikaler Knochenschrauben zu befestigen.

Aufgrund der besonderen Ausrichtung der Durchgangsöffnungen ist es dem Chirurgen möglich, ohne das Erfordernis eines transbuccalen Zugangs eine Osteosyntheseplatte intraoral zu platzieren, erforderlichenfalls Vorbohrungen vorzunehmen und anschließend die Osteosyntheseplatte mittels mehrerer Knochenschrauben zu befestigen. Zum Durchführen dieser Schritte genügen herkömmliche (langgestreckte) gerade Instrumente wie Klingen und Bohrer. Auf abgewinkelte Instrumente kann verzichtet werden. Ein weiterer Vorteil der in den Ausführungsbeispielen angegebenen Ausrichtung der Durchgangsöffnungen ist die Tatsache, dass der Chirurg trotz des intraoralen Zugangs ein gutes Sichtfeld hat und somit genau sehen kann, wo er bohrt und wo die Knochenschrauben platziert werden.

Obwohl die Erfindung anhand mehrerer Ausführungsbeispiele von Osteosyntheseplatten für die Versorgung von Kieferfrakturen erläutert wurde, eignen sich die erfindungsgemäßen Osteosyntheseplatten auch zur minimalinvasiven Versorgung anderer Frakturen im Kopfbereich (z.B. im Gesicht).

Aufgrund der obigen Erläuterungen werden dem Fachmann zahlreiche Änderungen, Ergänzungen und Weiterbildungen in den Sinn kommen, die von der Erfindung mit umfasst sind. Der Schutzbereich der Erfindung ist einzig durch die beigefügten Patentansprüche beschränkt.

## Patentansprüche

1. Osteosyntheseplatte (10) insbesondere zur Versorgung von Kieferfrakturen, mit
- einer Plattenebene;
- einem sich im Wesentlichen innerhalb der Plattenebene erstreckenden linearen ersten Abschnitt (12) mit einer ersten Längsachse (16); und
- einem sich im Wesentlichen innerhalb der Plattenebene schräg zum ersten Abschnitt (12) erstreckenden linearen zweiten Abschnitt (14) mit einer zweiten Längsachse (18),
**dadurch gekennzeichnet, dass** die Osteosyntheseplatte (10) umfasst:
- wenigstens eine kreisrunde erste Durchgangsöffnung (20) im ersten Abschnitt (12), die schräg zur Plattenebene verläuft und bezüglich der ersten Längsachse (16) eine erste Winkelausrichtung innerhalb der Plattenebene aufweist; und
- wenigstens eine kreisrunde zweite Durchgangsöffnung (22) im zweiten Abschnitt (14), die schräg zur Plattenebene verläuft und bezüglich der ersten Längsachse (16) des ersten Abschnitts (12) eine zweite Winkelausrichtung innerhalb der Plattenebene aufweist, wobei die erste und die zweite Winkelausrichtung bezüglich der ersten Längsachse (16) um weniger als ungefähr 60° voneinander abweichen, so dass Befestigungselemente (48) in einer durch einen einzigen intraoralen Zugang vorgegebenen Richtung in die Durchgangsöffnungen (20, 22) beider Plattenabschnitte (12, 14) einführbar sind.

2. Osteosyntheseplatte nach Anspruch 1, **dadurch gekennzeichnet, dass** die erste und die zweite Winkelausrichtung bezüglich der ersten Längsachse (16) um weniger als ungefähr 45° voneinander abweichen.

3. Osteosyntheseplatte nach Anspruch 1 oder 2, **dadurch gekennzeichnet, dass** die erste Winkelausrichtung schräg zur ersten Längsachse (16) und/oder die zweite Winkelausrichtung schräg zur zweiten Längsachse (18) verläuft.

4. Osteosyntheseplatte nach einem der Ansprüche 1 bis 3, **dadurch gekennzeichnet, dass** die wenigstens eine erste Durchgangsöffnung (20) die Plattenebene unter einem Winkel von ungefähr 20° bis 80° schneidet.

5. Osteosyntheseplatte nach einem der Ansprüche 1 bis 4, **dadurch gekennzeichnet, dass** die wenigstens eine zweite Durchgangsöffnung (22) die Plattenebene unter einem Winkel von ungefähr 20° bis 80° schneidet.

6. Osteosyntheseplatte nach einem der Ansprüche 1 bis 5, **dadurch gekennzeichnet, dass** die erste Winkelausrichtung bezüglich der ersten Längsachse (16) zwischen ungefähr +90° und -90° beträgt.

7. Osteosyntheseplatte nach einem der Ansprüche 1 bis 6, **dadurch gekennzeichnet, dass** die zweite Winkelausrichtung bezüglich der zweiten Längsachse (18) zwischen ungefähr 60° und 180° beträgt.

8. Osteosyntheseplatte nach einem der Ansprüche 1 bis 7, **dadurch gekennzeichnet, dass** die zweite Winkelausrichtung bezüglich der zweiten Längsachse (18) zwischen ungefähr 180° und 300° beträgt.

9. Osteosyntheseplatte nach einem der Ansprüche 1 bis 8, **dadurch gekennzeichnet, dass** der erste Abschnitt (12) und der zweite Abschnitt (14) unmittelbar aneinander grenzen.

10. Osteosyntheseplatte nach einem der Ansprüche 1 bis 9, **dadurch gekennzeichnet, dass** der erste Abschnitt (12) einen Winkel von ungefähr 90 bis 160° bezüglich des zweiten Abschnitts (14) aufweist.

11. Osteosyntheseplatte nach einem der Ansprüche 1 bis 10, **dadurch gekennzeichnet, dass** die Osteosyntheseplatte (10) wenigstens einen Biegebereich (30, 32, 40) von reduzierter Plattenstärke und/oder von reduzierter Plattenbreite und/oder von mäandernder Form aufweist.

12. Osteosyntheseplatte nach einem der Ansprüche 1 bis 11, **dadurch gekennzeichnet, dass** der erste Abschnitt (12) eine Länge zwischen ungefähr 5 und 70 mm aufweist und/oder der zweite Abschnitt (14) eine Länge zwischen ungefähr 5 und 70 mm aufweist.

13. Osteosyntheseplatte nach einem der Ansprüche 1 bis 12, **dadurch gekennzeichnet, dass** die Osteosyntheseplatte (10) im Bereich des ersten Abschnitts (12) und/oder im Bereich des zweiten Abschnitts (14) eine maximale Plattenstärke zwischen ungefähr 0,5 und 3,5 mm aufweist.

14. Osteosyntheseplatte nach einem der Ansprüche 1 bis 13, **dadurch gekennzeichnet, dass** die wenigstens eine erste Durchgangsöffnung (20) und/oder die wenigstens eine zweite Durchgangsöffnung (22) unterhalb einer Plattenoberfläche (28) eine Anschlag (26) für einen Kopf eines Befestigungselements aufweist.

15. Osteosyntheseplatte nach einem der Ansprüche 1 bis 14, **dadurch gekennzeichnet, dass** eine Mehrzahl erster Durchgangsöffnungen (20) und/oder eine Mehrzahl zweiter Durchgangsöffnungen (22) vorgesehen sind.

## Claims

1. A osteosynthesis plate (10), in particular for treating jaw fractures, with
- a plane of the plate;
- a linear first section (12) with a first longitudinal axis (16) and extending substantially within the plane of the plate; and
- a linear second section (14) with a second longitudinal axis (18) and extending substantially within the plane of the plate and inclined to the first section (12), **characterized in that** the osteosynthesis plate (10) comprises:
- at least one circular first through opening (20) in the first section (12), which is inclined to the plane of the plate and has with respect to the first longitudinal axis (16) a first angular orientation within the plane of the plate; and
- at least one circular second through opening (22) in the second section (14), which is inclined to the plane of the plate and has with respect to the first longitudinal axis (16) of the first section (12) a second angular orientation within the plane of the plate, wherein the first and the second angular orientations differ with respect to the first longitudinal axis (16) from one another by less than about 60°, so that fastening elements (48) can be introduced into the through openings (20, 22) of both plate sections (12, 14) in a direction predetermined by a single intraoral access.

2. The osteosynthesis plate according to claim 1, **characterized in that** the first and the second angular orientations differ with respect to the first longitudinal axis (16) from one another by less than about 45°.

3. The osteosynthesis plate according to claim 1 or 2, **characterized in that** the first angular orientation is inclined to the first longitudinal axis (16) and/or the second angular orientation is inclined to the second longitudinal axis (18).

4. The osteosynthesis plate according to one of claims 1 to 3, **characterized in that** the at least one first through opening (20) intersects the plane of the plate at an angle of approximately 20° to 80°.

5. The osteosynthesis plate according to one of claims 1 to 4, **characterized in that** the at least one second through opening (22) intersects the plane of the plate at an angle of approximately 20° to 80°.

6. The osteosynthesis plate according to one of claims 1 to 5, **characterized in that** the first angular orientation with respect to the first longitudinal axis (16) is between approximately +90° and -90°.

7. The osteosynthesis plate according to one of claims 1 to 6, **characterized in that** the second angular orientation with respect to the second longitudinal axis (18) is between approximately 60° and 180°.

8. The osteosynthesis plate according to one of claims 1 to 7, **characterized in that** the second angular orientation with respect to the second longitudinal axis (18) is between approximately 180° and 300°.

9. The osteosynthesis plate according to one of claims 1 to 8, **characterized in that** the first section (12) and the second section (14) directly adjoin one another.

10. The osteosynthesis plate according to one of claims 1 to 9, **characterized in that** the first section (12) has an angle of approximately 90° to 160° with respect to the second section (14).

11. The osteosynthesis plate according to one of claims 1 to 10, **characterized in that** the osteosynthesis plate (10) comprises at least one bending region (30, 32, 40) of reduced plate thickness and/or of reduced plate width and/or of meandering shape.

12. The osteosynthesis plate according to one of claims 1 to 11, **characterized in that** the first section (12) has a length between approximately 5 and 70 mm and/or the second section (14) has a length between approximately 5 and 70 mm.

13. The osteosynthesis plate according to one of claims 1 to 12, **characterized in that** the osteosynthesis plate (10) in the region of the first section (12) and/or in the region of the second section (14) has a maximum plate thickness between approximately 0.5 and 3.5 mm.

14. The osteosynthesis plate according to one of claims 1 to 13, **characterized in that** the at least one first through opening (20) and/or the at least one second through opening (22) has underneath a plate surface (28) a stop means (26) for a head of a fastening element.

15. The osteosynthesis plate according to one of claims 1 to 14, **characterized in that** a plurality of first through openings (20) and/or a plurality of second through openings (22) are provided.

## Revendications

1. Plaque d'ostéosynthèse (10), en particulier pour le traitement de fractures de mâchoires, comprenant
- un plan de plaque,
- un premier segment linéaire (12) qui s'étend pour l'essentiel dans le plan de la plaque et qui comporte un premier axe longitudinal (16), et
- un deuxième segment linéaire (14) qui s'étend pour l'essentiel dans le plan de la plaque, de manière oblique par rapport audit premier segment (12) et qui comporte un deuxième axe longitudinal (18),
**caractérisée en ce que** la plaque d'ostéosynthèse (10) comporte :
- au moins une première ouverture circulaire traversante (20) ménagée dans le premier segment (12), laquelle s'étend de manière oblique par rapport au plan de la plaque et présente une première orientation angulaire dans le plan de la plaque par rapport au premier axe longitudinal (16), et
- au moins une deuxième ouverture circulaire traversante (22) ménagée dans le deuxième segment (14), laquelle s'étend de manière oblique par rapport au plan de la plaque et présente une deuxième orientation angulaire dans le plan de la plaque par rapport au premier axe longitudinal (16) du premier segment (12), la première et la deuxième orientation angulaire divergeant l'une de l'autre de moins qu'environ 60° par rapport au premier axe longitudinal (16) si bien que des éléments de fixation (48) peuvent être introduits dans les ouvertures traversantes (20, 22) des deux segments de plaque (12, 14), dans une direction déterminée par un seul accès intra-oral.

2. Plaque d'ostéosynthèse selon la revendication 1, **caractérisé en ce que** la première et la deuxième orientation angulaire divergent l'une de l'autre de moins qu'environ 45° par rapport au premier axe longitudinal (16).

3. Plaque d'ostéosynthèse selon la revendication 1 ou 2, **caractérisée en ce que** la première orientation angulaire s'étend de manière oblique par rapport au premier axe longitudinal (16) et/ou la deuxième orientation angulaire s'étend de manière oblique par rapport au deuxième axe longitudinal (18).

4. Plaque d'ostéosynthèse selon l'une des revendications 1 à 3, **caractérisée en ce que** ladite première ouverture traversante (20) coupe le plan de la plaque sous un angle compris entre environ 20° et 80°.

5. Plaque d'ostéosynthèse selon l'une des revendications 1 à 4, **caractérisée en ce que** ladite deuxième ouverture traversante (22) coupe le plan de la plaque sous un angle compris entre 20° et 80°.

6. Plaque d'ostéosynthèse selon l'une des revendications 1 à 5, **caractérisée en ce que** la première orientation angulaire par rapport au premier axe longitudinal (16) est comprise entre environ +90° et -90°.

7. Plaque d'ostéosynthèse selon l'une des revendications 1 à 6, **caractérisée en ce que** la deuxième orientation angulaire par rapport au deuxième axe longitudinal (18) est comprise entre environ 60° et 180°.

8. Plaque d'ostéosynthèse selon l'une des revendications 1 à 7, **caractérisée en ce que** la deuxième orientation angulaire par rapport au deuxième axe longitudinal (18) est comprise entre environ 180° et 300°.

9. Plaque d'ostéosynthèse selon l'une des revendications 1 à 8, **caractérisée en ce que** le premier segment (12) et le deuxième segment (14) sont directement contigus.

10. Plaque d'ostéosynthèse selon l'une des revendications 1 à 9, **caractérisée en ce que** le premier segment (12) présente un angle compris entre enviro 90° et 160° par rapport au deuxième segment (14).

11. Plaque d'ostéosynthèse selon l'une des revendications 1 à 10, **caractérisée en ce que** la plaque d'ostéosynthèse (10) présente au moins une zone de pliage (30, 32, 40) d'une épaisseur de plaque réduite et/ou d'une largeur de plaque réduite et/ou d'une forme méandreuse.

12. Plaque d'ostéosynthèse selon l'une des revendications 1 à 11, **caractérisée en ce que** le premier segment (12) présente une longueur comprise entre environ 5, et 70 mm et/ou le deuxième segment (14), une longueur comprise entre environ 5 et 70 mm.

13. Plaque d'ostéosynthèse selon l'une des revendications 1 à 12, **caractérisée en ce que** la plaque d'ostéosynthèse (10) présente dans la zone du premier segment (12) et/ou dans la zone du deuxième segment (14) une épaisseur de plaque maximale comprise entre environ 0,5 et 3,5 mm.

14. Plaque d'ostéosynthèse selon l'une des revendications 1 à 13, **caractérisée en ce que** ladite première ouverture traversante (20) et/ou ladite deuxième ouverture traversante (22) présentent en dessous d'une surface de plaque (28) une butée (26) pour une tête d'un élément de fixation.

15. Plaque d'ostéosynthèse selon l'une des revendications 1 à 14, **caractérisée en ce que** sont prévues plusieurs premières ouvertures traversantes (20) et/ou plusieurs deuxièmes ouvertures traversantes (22).
